(19) Europäisches Patentamt
European Patent Office
Office européen des brevets

(11) Publication number : **0 535 704 A1**

# (12) EUROPEAN PATENT APPLICATION

(21) Application number : **92116922.3**

(51) Int. Cl.$^5$ : **A61K 9/02,** A61K 47/26

(22) Date of filing : **02.10.92**

(30) Priority : **04.10.91 JP 284231/91**

(43) Date of publication of application :
**07.04.93 Bulletin 93/14**

(84) Designated Contracting States :
**AT BE CH DE DK ES FR GB GR IE IT LI LU MC NL PT SE**

(71) Applicant : **SANWA KAGAKU KENKYUSHO CO., LTD.**
**No. 35, Higashi-sotobori-cho**
**Higashi-ku Nagoya-shi Aichi-ken (JP)**

(72) Inventor : **Kurono, Masayasu, c/o Sanwa Kagaku Kenkyusho**
**Co., Ltd., 35, Higashi-sotobori-cho, Higashi-ku Nagoya, Aichi-ken (JP)**
Inventor : **Kondo, Yasuaki, c/o Sanwa Kagaku Kenkyusho**
**Co., Ltd., 35, Higashi-sotobori-cho, Higashi-ku Nagoya, Aichi-ken (JP)**
Inventor : **Inoue, Tsuneaki, c/o Sanwa Kagaku Kenkyusho**
**Co., Ltd., 35, Higashi-sotobori-cho, Higashi-ku Nagoya, Aichi-ken (JP)**
Inventor : **Sato, Makoto, c/o Sanwa Kagaku Kenkyusho**
**Co., Ltd., 35, Higashi-sotobori-cho, Higashi-ku Nagoya, Aichi-ken (JP)**
Inventor : **Sugimoto, Manabu, c/o Sanwa Kagaku Kenkyusho**
**Co., Ltd., 35, Higashi-sotobori-cho, Higashi-ku Nagoya, Aichi-ken (JP)**
Inventor : **Noda, Hitoshi, c/o Sanwa Kagaku Kenkyusho**
**Co., Ltd., 35, Higashi-sotobori-cho, Higashi-ku Nagoya, Aichi-ken (JP)**
Inventor : **Ishida, Tsutomu, c/o Sanwa Kagaku Kenkyusho**
**Co., Ltd., 35, Higashi-sotobori-cho, Higashi-ku Nagoya, Aichi-ken (JP)**
Inventor : **Ito, Mayumi, c/o Sanwa Kagaku Kenkyusho**
**Co., Ltd., 35, Higashi-sotobori-cho, Higashi-ku Nagoya, Aichi-ken (JP)**
Inventor : **Goto, Yoshihito, c/o Sanwa Kagaku Kenkyusho**
**Co., Ltd., 35, Higashi-sotobori-cho, Higashi-ku Nagoya, Aichi-ken (JP)**
Inventor : **Sawai, Kiichi, c/o Sanwa Kagaku Kenkyusho**
**Co., Ltd., 35, Higashi-sotobori-cho, Higashi-ku Nagoya, Aichi-ken (JP)**

(74) Representative : **Wächtershäuser, Günter, Dr.**
**Tal 29**
**W-8000 München 2 (DE)**

## (54) Glycyrrhizin suppository.

(57) There is provided a glycyrrhizin suppository which is improved in drug absorptiveness from rectal wall and maintaining a drug concentration in blood at relatively high level over a prolonged period of time. The glycyrrhizin suppository comprises a compound selected from glycyrrhizic acid and salts thereof, and a non-ionic surface active agent, as indispensable component, in addition to an oleaginous base. The suppository may contain a compound selected from water soluble carboxylic acids and salts thereof.

EP 0 535 704 A1

The present invention relates to a glycyrrhizin suppository improved in its drug absorptiveness and maintaining a drug concentration in blood at relatively high level over a prolonged period of time.

As having been known, glycyrrhizic acid and its salt have several pharmacological actions such as an anti-cortisonic action, cholesterol lowering action, anti-allergic action, anti-inflammatory action, antidotal action, gastric ulcer repair action, and the like. However, a glycyrrhizin pharmaceutical preparation containing the glycyrrhizic acid or its salt as an effective ingredient has been mainly put to practical use as a medicine for curing hepatic diseases.

In general, regardless of acute or chronic diseases, the hepatic must be continuously administrated over relatively long period of time. In the case of the glycyrrhizin pharmaceutical preparation, it shall be administrated as an injection every day for an in-patient, whereas 3 times in a week to an out-patient. For the out-patient, a solid medicine formulated for oral dosage shall be given for compensating a lack of dose to administrate the same, when he will not come to the hospital. Although the glycyrrhizin preparation for oral dosage was developed to make patient decrease the frequency attending to the hospital and make light his burden, as a matter of fact, the oral preparation has been doubted on its pharmacological effect compared with the injectional administration.

In case where a systemic action by a medicine is expected, the oral administration of medicine is easily affected by absorption inhibition factor by way passing through a digestive canal, and thus an administration into rectum shall be considered as one of excellent administration routes to avoid the influence of the absorption inhibition factor.

Regarding the administration into the rectum, it has already been reported that in case of the glycyrrhizin preparation formed by combining the drug of glycyrrhizic acid or a salt thereof to an oleaginous base, the drug is absorbed well from the rectal wall, an absorption ratio is increased remarkably and a biological availability can be improved, in comparison with those on oral administration [Jap. Pat. Nos. Hei 1 (A.D. 1989) - 294619(A), 3 (A.D. 1991) - 2122(A) and 3 (A.D. 1991) - 123731(A)]. However, the suppository described in these publications cannot gain same concentration level in blood with that in the injection capable of 100%, and thereby the absorption of drug in the form of suppository should be improved.

As a general information for improving an absorptiveness of certain drugs, it has been reported to add an absorption promoter such as a non-ionic surface active agent, sodium caprate and abilate [Noboru YATA "Pharm. Tech. Japan", Vol. 4, page 282 (1988)], but there is issued no report of that an absorption promoter was applied to the glycyrrhizin preparation.

Therefore, the inventors have energetically studied and investigated to apply various absorption promoters for a glycyrrhizin suppository to find that feces remained in the rectum affected to the drug to change an amount of drug absorption between individuals in very wide range, so that a drug amount should be set in higher level to bring its concentration in blood to same with the level in the case of injectional administration. If large amount of the drug shall be administrated into the rectum, it is suspected that a side effect of pseudo-aldosteronism will be revealed as well as in excessive administration on oral dosage, although the amount of drug absorption in glycyrrhizin suppository would be remarkably increased than that of the oral administration.

Then, the inventors have further studied for developing a glycyrrhizin suppository which expresses stabilized pharmacological effect by administration of relatively small amount of the drug and shows high drug concentration in blood equivalent to widely used glycyrrhizin injection.

As a result, they have found that a glycyrrhizin suppository which was prepared by adding at least one kind of compounds selected from glycyrrhizic acid and non-toxic salts thereof, as an effective ingredient, a non-ionic surface active agent as an absorption promoter and adding, if necessary, a compound selected from water soluble carboxylic acids and non-toxic salts thereof, and composing the ingredients into a conventional base for suppositories can be remarkably improved in its absorptiveness, decreases a deviation in absorption amount between individuals, and prolongs its absorption period of time, when it compared with a conventional glycyrrhizin suppository to be prepared by mixing the drug with an oleaginous base, to establish the invention.

When the glycyrrhizin suppository according to the invention is administrated into rectum, the absorption rate of suppository is much higher than that of the conventional suppository as disclosed in said Jap. Pat. No. Hei 1 (A.D. 1989) - 294619(A), and thus if the same pharmacological effect shall be expected, an amount of the drug can be reduced to suppress an occasion of the side effect, and an excellent pharmaceutical effect compatible with the conventional injectional administration can be expected.

As the non-ionic surface active agent for the production of the glycyrrhizin suppository according to the invention, those are desirable which do not show a reactivity with glycyrrhizic acid and its salt, has low stimulation to rectum and has HLB value (HLB : Hydrophile-lypophile balance) of 8 - 20, and more preferably in the rage of 11 - 18. On the other hand, an amount of the surface active agent to be added is preferably 0.1 - 20% by weight, and more preferably 1 - 10% by weight. Because the absorptiveness of drug is not satisfactory at the time of administration, when the surface active agent having HLB value of 8 or less is used, or when the added amount of the surface active agent is 0.1% by weight or less. If the adding amount of surface active agent is more than 20% by weight, it is anxious about the stimulation to the rectal mucosa.

Following non-ionic surface active agents can be listed: polyoxyethylene alkyl ether [such as "Nikkole BL-9EX- BC-20TX" produced by Nikko Chemicals Co., Ltd.], polyethylene-glycol fatty acid ester [such as "Nikkole MYS-40, MYO-10" produced by Nikko Chemicals Co., Ltd.], polyoxyethylene alkylphenyl ether [such as "Nikkole OP-10, NP-15" produced by Nikko Chemicals Co., Ltd.], polyoxyethylene sorbitan fatty acid ester [such as "P-1570", "L-1695" produced by Mitsubishi Chemical Industries Ltd.]. These can be used solely or in any combination.

It is important that the water soluble carboxylic acid or its salt does not show reactivity with the glycyrrhizic acid, its salt and the base for the suppository, and shows low stimulation to the rectum. A particle size thereof is preferably 150μ m or less, in view point of its dispersion, when the suppository shall be prepared. It is preferable to add the water soluble carboxylic acid and/or its salt in a range of 1 - 60% by weight, and more preferably 10 - 40% by weight, because the absorption improving effect is not satisfied in 1% by weight or less and because the dispersion thereof becomes difficult in 60% by weight or more.

The carboxylic acid and its salt include malonic acid, succinic acid, maleic acid, fumaric acid, phtalic acid, capric acid, capronic acid, tartalic acid, malic acid, citric acid and its alkaline metal salts, basic amine salts, bissodium ethylene diamine tetraacetate, nitrilo triacetate, bissodium cyclohexane diamine tetraacetate, and the like. These are used solely or in any combination.

The base for suppository includes oleaginous bases [e.g. "Witepsol" (trademark) produced by Huels Troisdorf A.G.], "Pharmasol" (trademark) produced by Nippon Oil & Fats Co., Ltd., "Isocacao" (trademark) produced by Kao Co., Ltd.] and water soluble bases [e.g. polyethylene glycol etc.]. These are used solely or in any combination.

When the suppository according to the the invention poured into water at 37°C and stirred to completely dissolve the same, pH of water layer shows a value between 5.5 - 8.0. If the pH shows less than 5.5 or higher than 8.0, it can be recognized that an absorptiveness of,such suppository becomes low. In such a case, an agent for adjusting pH may be added to set the pH into said preferable range.

There is no particular limitation in production procedures for the suppository according to the invention, and thus it can be prepared in a conventional manner. For instance, the suppository can be prepared by adding the non-ionic surface active agent to a glyceride, heating the same to cause'melting thereof, adding the water soluble carboxylic acid or its salt to mix the same, adding the glycyrrhizic acid or its salt as the effective ingredient to stir the same for preparing a homogenous mixture, and then pouring the mixture into a mold for formation and solidification. If necessary, a dispersing agent preservative, stabilizer, coloring agent and the like may be added at a suitable step for preparing the suppository.

This invention will now be further explained in more detail with reference to Reference Examples, Examples and Test Examples which shall refer to single drawing, wherein

Fig. 1 is a graph showing results of researching a transition in concentration of glycyrrhizic acid in plasma, when a suppository prepared by Reference Examples and Examples was administrated into rectum of a beagle dog and injectionally obtained blood with the passage of time.

Reference Example 1

Ingredients :

| | |
|---|---|
| Dipotassium glycyrrhizinate | 250 (mg) |
| Witepsol W-35 | 1250 |
| | Total 1500 mg/piece |

To melted Witepsol W-35 by heating the same at 50°C , bipotassium glycyrrhizinate was added and stirred to mix the same. The mixture was left to stand until its temperature becomes 40 - 45°C . Then, the mixture was charged into a mold which was cooled at 25°C in water-bath to cause a solidification of the content to obtain a suppository.

Reference Example 2

| | | |
|---|---|---|
| Component A : | | |
| | Dipotassium glycyrrhizinate | 250 (mg) |
| | L-Arginine | 82 |
| | Trisodium citrate | 500 |
| Component B : | | |
| | Nikkole BL-9EX | 45 (mg) |
| | Witepsol S-55 | 663 |
| | | Total 1500 mg/piece |

To the component B heated and melted at 50°C , the component A was added and sufficiently stirred to mix the same. The mixture was left to stand for cooling to 40 - 45°C and then charged into a mold which was cooled at 25°C by water-bath to cause a solidification of the content to obtain a suppository.

Example 1

| | | |
|---|---|---|
| Component A : | | |
| | Dipotassium glycyrrhizinate | 250 mg |
| B component : | | |
| | Nikkole BL-9EX | 45 (mg) |
| | Witepsol S-55 | 1205 |
| | | Total 1500 mg/piece |

To the component B heated and melted at 50°C , the component A was added and sufficiently stirred to mix the same. The mixture was left to stand for cooling to 40 - 45°C and then charged into a mold which was cooled at 25°C by water-bath to cause a solidification of the content to obtain a suppository.

Example 2

| | | |
|---|---|---|
| Component A : | | |
| | Dipotassium glycyrrhizinate | 250 (mg) |
| | Trisodium citrate | 500 |
| Component B : | | |
| | Nikkole BL-9EX | 45 (mg) |
| | Witepsol W-35 | 705 |
| | | Total 1500 mg/piece |

With use of the both components, a suppository was prepared as described in Example 1.

Example 3

| | | |
|---|---|---|
| Component A : | | |
| | Dipotassium glycyrrhizinate | 250 (mg) |
| | Disodium citrate | 500 |
| Component B : | | |
| | Nikkole MYS-40 | 45 (mg) |
| | Witepsol W-55 | 705 |
| | | Total 1500 mg/piece |

With use of the both components, a suppository was prepared as described in Example 1.

Example 4

| | | |
|---|---|---|
| Component A : | | |
| | Diammonium glycyrrhizinate | 250 (mg) |
| | Ryoto-sugar ester (P-1570) | 75 |
| | Bipotassium tartarate | 300 |
| Component : | | |
| | Witepsol S-55 | 875 mg |
| | | Total 1500 mg/piece |

With use of the both components, a suppository was prepared as described in Example 1.

Example 5

| | | |
|---|---|---|
| Component A : | | |
| | Diammonium glycyrrhizinate | 250 (mg) |
| | Diammonium ethylene diamine tetraacetate (powderized substance, after adjusted its pH to 7) | 500 |
| Component B : | | |
| | Nikkole TO-10M | 45 (mg) |
| | Witepsol H-15 | 705 |
| | | Total 1500 mg/piece |

With use of the both components, a suppository was prepared as described in Example 1.

Example 6

| | | |
|---|---|---|
| Component A : | | |
| | Glycyrrhizic acid | 250 (mg) |
| | Sodium malate | 500 |
| | L-Lysine | 95 |
| Component B : | | |
| | Nikkole HCO-60 | 45 (mg) |
| | Witepsol W-35 | 610 |
| | | Total 1500 mg/piece |

With use of the both components, a suppository was prepared as described in Example 1.

Test Example 1

Each suppository obtained by the Reference Examples and Examples was poured into a flask accommodating water kept at 37°C by water-bath to shake for 2 hours, and water layer was taken to measure pH value thereof.

Results are shown in following Table 1. The values were 8 or less except for the suppository obtained by Reference Example 2.

Table 1

| Suppositories | pH values |
|---|---|
| Reference Example | |
| 1 | 5.2 |
| 2 | 8.3 |
| Example | |
| 1 | 5.3 |
| 2 | 6.3 |
| 3 | 5.5 |
| 4 | 6.2 |
| 5 | 5.8 |
| 6 | 6.8 |

Test Example 2

Into each flask, added feces (10g) of a beagle dog, saline (20ml), and the suppository obtained by one of Reference Examples or Examples to left to stand for 2 hours at 37°C . Then, each of the flask was shaken for 30 minutes and centrifuged at 3000 rpm for 10 minutes to obtain a supernatant. An amount of glycyrrhizic acid was measured by the method of HPLC, and a rate of glycyrrhizic acid incorporated into the feces was calculated.

Results are shown in following Table 2. It has been difinitely shown that the amount of glycyrrhizic acid was decreased, when the non-ionic surface active agent was combined with the water soluble carboxylic acid or carboxylate, and pH value was adjusted 5,5 or more. These results suggests that the amount of glycyrrhizic acid incorporated into the feces can be decreased and that an absolute amount of glycyrrhizic acid absorbed from the rectal wall, even if residuary feces exists in the rectum.

Table 2

| Suppositories | Rates (%) of glycyrrhizic acid incorporated into feces |
|---|---|
| Reference Example | 58.3 |
| 1 | |
| 2 | 24.2 |
| Example | |
| 1 | 51.8 |
| 2 | 30.5 |
| 3 | 31.3 |
| 4 | 39.1 |
| 5 | 33.4 |
| 6 | 29.5 |

Test Example 3

Each of the suppositories obtained by Reference Examples 1 and 2 as well as Examples 1, 2, 4 and 6 was administrated into the rectum of beagle dogs fasted for 24 hours, and the blood was injectionally obtained from its front leg at 0.5, 1, 2, 4, 6, 8 and 24 hours after the administration of suppository to obtain plasma therefrom by a conventional method. Concentration of glycyrrhizic acid in the plasma was measured by the method of HPLC to calculate the $AUC_{0-24}$ (AUC : area under the curve) based on the concentrations of glycyrrhizic acid in the plasma sample.

Results are shown in following Table 3. The $AUC_{0-24}$ value on the suppository obtained by Example 1 is extremely higher than that on the suppository obtained by Reference Example 1. The $AUC_{0-24}$ values on the suppositories obtained by Examples 2, 4 and 6, wherein carboxylic acid or its salt was added and pH was adjusted, were higher than that on the suppository obtained by Example 1, and the change between individuals came to small.

Change or transition of glycyrrhizic acid concentration with the passage of time are shown in Fig. 1. The maximum drug concentration in plasma on the suppository obtained by Reference Example 1 was 7 nm (nanomoles)/ml or less. On the contrary thereto, maximum drug concentration on the suppository obtained by Example 1 was 40 nm/ml or more, and those obtained by Examples 2, 4 and 6 were 30 nm/ml or more, and the suppositories obtained by the Examples have maintained the concentration of relatively high level. Further, it has been found that the concentration of suppositories obtained by Examples 2, 4 and 6 had reached to maximum one by 4 - 6 hours from the administration, and thereafter showed a slow descent while maintaining relatively high drug concentration level, without cause any rapid decrease, on the contrary to the suppository obtained by Example 1. This fact shows that such suppositories develops its pharmacological action over a prolonged period of time.

The suppository obtained by Reference Example 2 was prepared so as to set its pH of 8.3 in the form of solution (see Test Example 1) and shows relatively good concentration level in blood, with the passage of time, but not better than that on the suppositories obtained by Examples 2, 4 and 6, and thereby it has been found that setting of pH to 8 or more is not preferable, since absorption of glycyrrhizic acid will decrease.

Table 3

| Suppositories | $AUC_{0-24}$ (nm · hr/ml) | Variation coefficient (%) |
|---|---|---|
| Reference Example | | |
| 1 | 116.7 | 9.8 |
| 2 | 568.2 | 44.0 |
| Example | | |
| 1 | 512.0 | 19.5 |
| 2 | 800.2 | 10.5 |
| 4 | 748.9 | 11.2 |
| 6 | 788.0 | 9.7 |

## Claims

**(1)** A glycyrrhizin suppository comprising at least one of active ingredients selected from the group consisting of glycyrrhizic acid and salts thereof, and at least one of non-ionic surface active agents, in addition to an oleaginous base for suppositories.

**(2)** A glycyrrhizin suppository as claimed in Claim 1, further comprising at least one of water soluble carboxylic acids and salts thereof.

**(3)** A glycyrrhizin suppository as claimed in Claim 1 or 2, wherein pH of water layer is in the range of 5.5 - 8.0, when the suppository is dissolved in water.

**(4)** A glycyrrhizin suppository as claimed in Claim 2, wherein an amount of said water soluble carboxylic acid or its salt is in the range of 1 - 60% by weight.

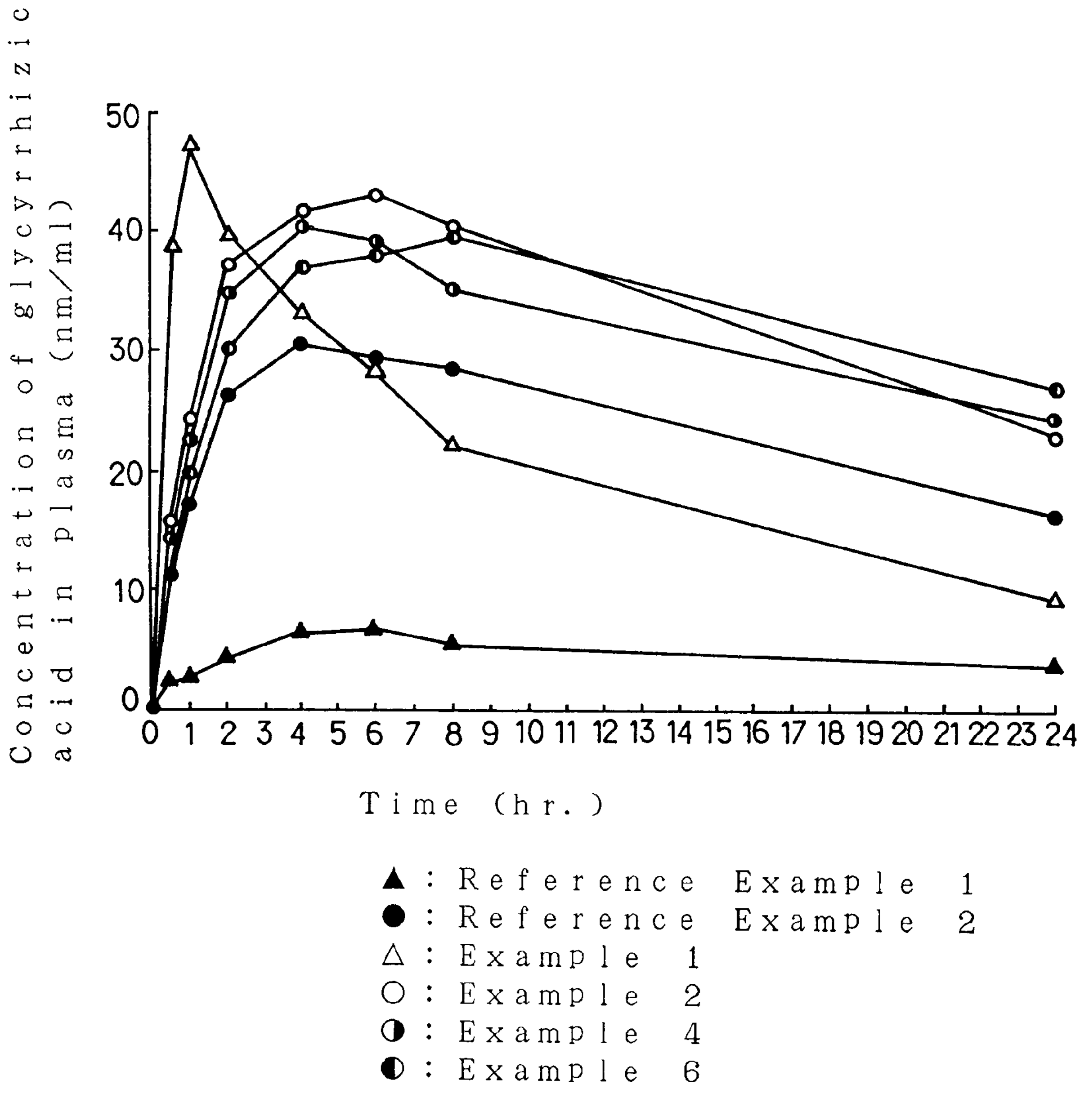
Concentration of glycyrrhizic acid in plasma (nm/ml)
50
40
30
20
10
0
0 1 2 3 4 5 6 7 8 9 10 11 12 13 14 15 16 17 18 19 20 21 22 23 24
Time (hr.)
▲ : Reference Example 1
● : Reference Example 2
△ : Example 1
○ : Example 2
◑ : Example 4
◐ : Example 6

European Patent Office

# EUROPEAN SEARCH REPORT

Application Number

EP 92 11 6922

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 397 447 (ISF SOCIETA PER AZIONI)<br>* page 3, line 23 - line 32 *<br>* page 4, line 28 - line 33 *<br>* page 4, line 53 - line 57 *<br>* page 5, line 28 - line 30 *<br>* examples 40-43 *<br>----- | 1-4 | A61K9/02<br>A61K47/26 |

TECHNICAL FIELDS SEARCHED (Int. Cl.5 )

A61K

The present search report has been drawn up for all claims

| Place of search | Date of completion of the search | Examiner |
|---|---|---|
| THE HAGUE | 12 NOVEMBER 1992 | VENTURA AMAT A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)